(19) Europäisches Patentamt
European Patent Office
Office européen des brevets

(11) **EP 2 272 910 B1**

(12) **EUROPEAN PATENT SPECIFICATION**

(45) Date of publication and mention
of the grant of the patent:
**12.03.2014 Bulletin 2014/11**

(51) Int Cl.:
***C08L 23/00*** *(2006.01)*

(21) Application number: **10158276.5**

(22) Date of filing: **25.08.2004**

(54) **Aqueous dispersion**

Waesserige dispersion

Dispersion aqueuse

(84) Designated Contracting States:
**AT BE BG CH CY CZ DE DK EE ES FI FR GB GR HU IE IT LI LU MC NL PL PT RO SE SI SK TR**

(30) Priority: **25.08.2003 US 497527 P**
**27.02.2004 US 548493 P**

(43) Date of publication of application:
**12.01.2011 Bulletin 2011/02**

(62) Document number(s) of the earlier application(s) in accordance with Art. 76 EPC:
**04782191.3 / 1 675 902**

(73) Proprietor: **Dow Global Technologies LLC**
**Midland, MI 48674 (US)**

(72) Inventors:
• **Moncla, Brad M.**
**Lake Jackson, TX 77566 (US)**
• **Kalinowski, Matthew J.**
**Freeland, MI 48623 (US)**
• **Speth, David R.**
**Upper Arlington, OH 43220 (US)**

• **Strandburg, Gary M.**
**Mount Pleasant, MI 48858 (US)**
• **Diehl, Charles**
**Blue Bell, PA 19422 (US)**
• **Schmidt, Dale**
**Midland, MI 48642 (US)**
• **Maak, Kevin**
**Midland, MI 48640 (US)**

(74) Representative: **Raynor, John**
**Beck Greener**
**Fulwood House**
**12 Fulwood Place**
**London**
**WC1V 6HR (GB)**

(56) References cited:
| | |
|---|---|
| **EP-A- 0 525 205** | **EP-A- 0 718 318** |
| **EP-A- 1 035 166** | **EP-A- 1 394 202** |
| **WO-A-01/82708** | **WO-A-02/064856** |
| **WO-A-03/025058** | **DE-A- 4 428 382** |
| **US-A- 4 689 351** | **US-A- 6 130 266** |
| **US-A1- 2003 158 341** | **US-B1- 6 339 123** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Aqueous dispersions of a thermoplastic resin of various types are known in the art. Aqueous dispersions have been used in a wide variety of fields since an aqueous dispersion prepared by using water as its dispersion medium is far more advantageous than the dispersions prepared by using an organic solvent for the dispersion medium in view of flammability, working environment, handling convenience, and the like. For example, when an aqueous dispersion is coated and dried on a surface of a substrate such as paper, fiber, wood, metal, or plastic molded article, the resin coating formed will provide the substrate with water resistance, oil resistance, chemical resistance, corrosion resistance and heat sealability.

Conventional aqueous dispersions of a thermoplastic resin have been produced either by a process wherein a polymerizable monomer which is the resin raw material is polymerized by emulsion polymerization in an aqueous medium in the presence of a dispersing agent, or by a process wherein a molten thermoplastic resin and an aqueous medium, and optionally, a dispersing agent are mixed by applying shearing force. The former process is associated with the disadvantage of the limited number of the polymerizable monomers that can be used, and hence, the variety of the aqueous dispersions of the thermoplastic resin that can be produced, is limited. The former process also suffers from complicated control of the polymerization reaction as well as intricate equipment. On the other hand, the latter process is applicable to a wide variety of resins in relatively simple equipment.

In one aspect the invention provides an aqueous dispersion comprising

(A) at least one thermoplastic resin;
(B) at least one dispersing agent comprising at least one carboxylic acid, a salt of at least one carboxylic acid, a carboxylic acid ester or salt of a carboxylic acid ester, or an ethylene acid polymer;
(C) water; and optionally
(D) a neutralizing agent selected from potassium hydroxide, sodium hydroxide and combinations thereof;

wherein the dispersion has a pH of from 7 to 11, and wherein the thermoplastic resin is selected from an alpha-olefin interpolymer of ethylene with at least one comonomer selected from propylene, 1-butene, 3-methyl-1-butene, 4-methyl-1-pentene, 3-methyl-1-pentene, 1-heptene, 1-hexene, 1-octene, 1-decene, and 1-dodecene, wherein the dispersion has a particle size distribution (defined as volume average particle diameter Dv divided by number average particle diameter Dn) of less than or equal to 2.0, and wherein the dispersing agent comprises less than about 4 percent by weight based on the weight of the thermoplastic resin.

The thermoplastic resin is an alpha-olefin interpolymer of ethylene with at least one comonomer selected from propylene, 1-butene, 3-methyl-1-butene, 4-methyl-1-pentene, 3-methyl-1-pentene, 1-heptene, 1-hexene, 1-octene, 1-decene, and 1-dodecene. In some embodiments, the interpolymer of ethylene has a density of less than about 0.92 g/cc.

In some embodiments, the at least one thermoplastic resin has a crystallinity of less than about 50 %. In other embodiments, the crystallinity ranges from about 5 % to about 45 %, or from about 5 % to about 40 %.

Any suitable dispersing agent can be used. However, in particular embodiments the dispersing agent comprises at least one carboxylic acid, a salt of at least one carboxylic acid, or carboxylic acid ester or salt of the carboxylic acid ester. One example of a carboxylic acid useful as a dispersant is a fatty acid such as montanic acid. In some preferred embodiments, the carboxylic acid, the salt of the carboxylic acid, or at least one carboxylic acid fragment of the carboxylic acid ester or at least one carboxylic acid fragment of the salt of the carboxylic acid ester has fewer than 25 carbon atoms. In other embodiments, the carboxylic acid, the salt of the carboxylic acid, or at least one carboxylic acid fragment of the carboxylic acid ester or at least one carboxylic acid fragment of the salt of the carboxylic acid ester has 12 to 25 carbon atoms. In some embodiments, carboxylic acids, salts of the carboxylic acid, at least one carboxylic acid fragment of the carboxylic acid ester or its salt has 15 to 25 carbon atoms are preferred. In other embodiments, the number of carbon atoms is 25 to 60. Some preferred salts comprise a cation selected from the group consisting of an alkali metal cation, alkaline earth metal cation, or ammonium or alkyl ammonium cation.

In still other embodiments, the dispersing agent is selected from the group consisting of ethylene carboxylic acid polymers, and their salts, such as ethylene acrylic acid copolymers or ethylene methacrylic acid copolymers.

In other embodiments, the dispersing agent is selected from alkyl ether carboxylates, petroleum sulfonates sulfonated polyoxyethylenated alcohol, sulfated or phosphated polyoxyethylenated alcohols, polymeric ethylene oxide/propylene oxide/ethylene oxide dispersing agents, primary and secondary alcohol ethoxylates, alkyl glycosides and alkyl glycerides.

Combinations any of the above-enumerated dispersing agents can also be used to prepare some aqueous dispersions.

Some dispersions described herein have an advantageous particle size distribution. In particular embodiments, the dispersion has a particle size distribution defined as volume average particle diameter (Dv) divided by number average particle diameter (Dn) of less than or equal to about 2.0. In other embodiments, the dispersion has a particle size distribution of less than or equal to about less than 1.5.

Some dispersions described herein comprise particles having an average particle size of less than about 1.5 μm. In other embodiments, the average particle size ranges from about 0.05 μm to about 1.5 μm. In still other embodiments, the average particle size of the dispersion ranges from about 0.5 μm to about 1.5 μm.

For dispersions having a pH of less than 12, some dispersions have a pH of from about 5 to about 11.5, preferably from about 7 to about 11, more preferably from about 9 to about 11. The pH can be controlled by a number of factors, including type or strength of base (dispersing agent), degree of conversion of the base to the salt form, type of thermoplastic polymer to be dispersed, and melt kneading (e.g., extruder) processing conditions. The pH can be adjusted either in-situ, or by converting the carboxylic acid dispersing agent to the salt form before adding it to the thermoplastic resin and forming the dispersion. Of these, forming the salt in-situ is preferred.

Preferably, the dispersions are characterized by a percent solids content of less than about 74 % by volume. Some dispersions have a percent solids of from about 5 % to about 74 % by volume. Still other dispersions have a percent solids of less than about 70 % by volume, less than about 65 % by volume, or from about 5 % to about 50 % by volume.

In another aspect embodiments of the invention provide a method for producing an aqueous dispersion comprising: (1) melt kneading (A) at least one thermoplastic resin and (B) at least one dispersing agent, to produce a melt-kneaded product and (2) diluting said melt-kneaded product, and melt kneading the resulting mixture to form the aqueous dispersion, wherein the dispersion has an average particle size of less than about 5 μm. Other embodiments provide a method for producing an aqueous dispersion comprising:(1) melt kneading (A) at least one thermoplastic resin and (B) at least one dispersing agent, to produce a melt-kneaded product and (2) diluting said melt-kneaded product, and melt kneading the resulting mixture to form the aqueous dispersion to produce a dispersion having a pH of less than 12. In some methods according to either aspect, the dispersing agent comprises less than about 4 percent by weight based on the weight of the thermoplastic resin. In some methods that provide a dispersion having a pH of 12 or less, the dispersion also has a volume average particle size of less than about 5 μm. Some dispersions that have a particle size of less than about 5 μm also have a pH of less than 12. Embodiments of the methods use the thermoplastic resins and dispersing agents described above. And in some embodiments, the methods provide dispersions having one or more of the properties described above.

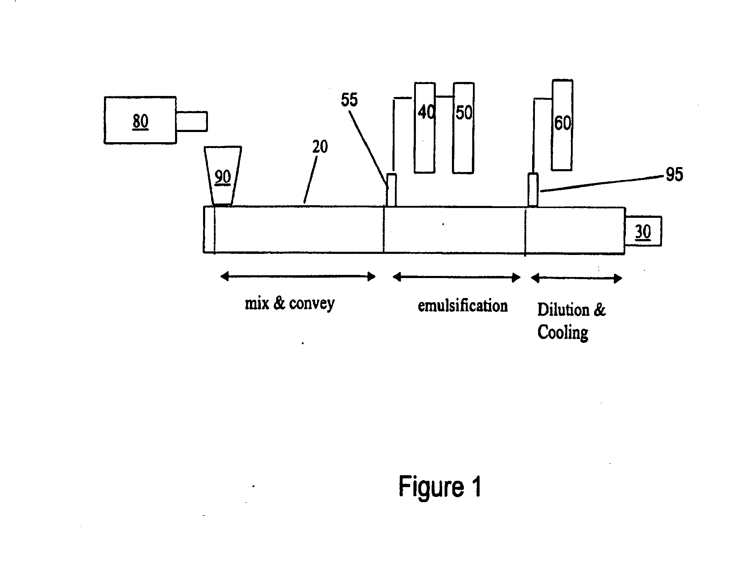

FIG. 1 is a schematic representation of a typical melt-extrusion apparatus used to prepare embodiments of the invention.

In the following description, all numbers disclosed herein are approximate values, regardless whether the word "about" or "approximate" is used in connection therewith. They may vary by 1 %, 2 %, 5 %, and sometimes, 10 to 20 %. Whenever a numerical range with a lower limit, RL and an upper limit, RU, is disclosed, any number falling within the range is specifically disclosed. In particular, the following numbers within the range are specifically disclosed: $R = R_L + k*(R_U - R_L)$, wherein k is a variable ranging from 1 % to 100 % with a 1 % increment, i.e., k is 1 %, 2 %, 3 %, 4 %, 5 %,..., 50 %, 51 %, 52 %,..., 95 %, 96 %, 97 %, 98 %, 99 %, or 100 %. Moreover, any numerical range defined by two R numbers as defined in the above is also specifically disclosed.

The thermoplastic resin (A) included in embodiments of the aqueous dispersion of the present invention is a resin that is not readily dispersible in water by itself. In some embodiments it is present in the dispersion in an amount of from greater than 0 percent by wt. to less than about 96 percent by wt. In certain embodiments, the resin is present in an amount of from about 35 to about 65 percent by wt. of the dispersion. The term "resin" used herein should be construed to include synthetic polymers or chemically modified natural resins such as but not limited to thermoplastic materials such as polyvinyl chloride, polystyrene, and polyethylene and thermosetting materials such as polyesters, epoxies, and silicones that are used with fillers, stabilizers, pigments, and other components to form plastics. The term resin as used herein includes elastomers and is understood to include blends of olefin polymers. In some embodiments, the thermoplastic resin is a semi-crystalline resin. The term "semi-crystalline" is intended to identify those resins that possess at least one endotherm when subjected to standard differential scanning calorimetry (DSC) evaluation. Some semi-crystalline polymers exhibit a DSC endotherm that exhibits a relatively gentle slope as the scanning temperature is increased past the final endotherm maximum. This reflects a polymer of broad melting range rather than a polymer having what is generally considered to be a sharp melting point. Some polymers useful in the dispersions of the invention have a single melting point while other polymers have more than one melting point. In some polymers one or more of the melting points may be sharp such that all or a portion of the polymer melts over a fairly narrow temperature range, such as a few degrees centigrade. In other embodiments, the polymer may exhibit broad melting characteristics over a range of about 20 °C.

The thermoplastic resin (A) is selected from an alpha-olefin interpolymer of ethylene with at least one comonomer selected from propylene, 1-butene, 3-methyl-1-butene, 4-methyl-1-pentene, 3-methyl-1-pentene, 1-heptene, 1-hexene, 1-octene, 1-decene, and 1-dodecene.

$^{13}$C NMR spectroscopy is one of a number of techniques known in the art of measuring comonomer incorporation into a polymer. An example of this technique is described for the determination of comonomer content for ethylene/α-olefin copolymers in Randall (Journal of Macromolecular Science, Reviews in Macromolecular Chemistry and Physics, C29 (2 & 3), 201-317 (1989)). The basic procedure for determining the comonomer content of an olefin interpolymer involves obtaining the $^{13}$C NMR spectrum under conditions where the intensity of the peaks corresponding to the different carbons in the sample is directly proportional to the total number of contributing nuclei in the sample. Methods for ensuring this proportionality are known in the art and involve allowance for sufficient time for relaxation after a pulse, the use of gated-decoupling techniques, relaxation agents, and the like. The relative intensity of a peak or group of peaks is obtained in practice from its computer-generated integral. After obtaining the spectrum and integrating the peaks, those peaks associated with the comonomer are assigned. This assignment can be made by reference to known-spectra or literature, or by synthesis and analysis of model compounds, or by the use of isotopically labelled comonomer. The mole % comonomer can be determined by the ratio of the integrals corresponding to the number of moles of comonomer to the integrals corresponding to the number of moles of all of the monomers in the interpolymer, as described in Randall, for example.

The data is collected using a Varian UNITY Plus 400 MHz NMR spectrometer, corresponding to a $^{13}$C resonance frequency of 100.4 MHz. Acquisition parameters are selected to ensure quantitative $^{13}$C data acquisition in the presence of the relaxation agent. The data is acquired using gated $^1$H decoupling, 4000 transients per data file, a 7 sec pulse repetition delay, spectral width of 24,200 Hz and a file size of 32 K data points, with the probe head heated to 130 °C. The sample is prepared by adding approximately 3 mL of a 50/50 mixture of tetrachloroethane-d2/orthodichlorobenzene that is 0.025 M in chromium acetylacetonate (relaxation agent) to 0.4 g sample in a 10 mm NMR tube. The headspace of the tube is purged of oxygen by displacement with pure nitrogen. The sample is dissolved and homogenized by heating the tube and its contents to 150 °C with periodic refluxing initiated by heat gun.

Molecular weight distribution of the polymers is determined using gel permeation chromatography (GPC) on a Polymer Laboratories PL-GPC-220 high temperature chromatographic unit equipped with four linear mixed bed columns (Polymer Laboratories (20-micron particle size)). The oven temperature is at 160 °C with the autosampler hot zone at 160 °C and the warm zone at 145 °C. The solvent is 1,2,4-trichlorobenzene containing 200 ppm 2,6-di-t-butyl-4-methylphenol. The flow rate is 1.0 milliliter/minute and the injection size is 100 microliters. About 0.2 % by weight solutions of the samples are prepared for injection by dissolving the sample in nitrogen purged 1,2,4-trichlorobenzene containing 200 ppm 2,6-di-t-butyl-4-methylphenol for 2.5 hrs at 160 °C with gentle mixing.

The molecular weight determination is deduced by using ten narrow molecular weight distribution polystyrene standards (from Polymer Laboratories, EasiCal PS1 ranging from 580 - 7,500,000 g/mole) in conjunction with their elution volumes. The equivalent propylene-ethylene copolymer molecular weights are determined by using appropriate Mark-Houwink coefficients for polypropylene (as described by Th.G. Scholte, N.L.J. Meijerink, H.M. Schoffeleers, and A.M.G. Brands, J. Appl. Polym. Sci., 29, 3763 - 3782 (1984)) and polystyrene (as described by E. P. Otocka, R. J. Roe, N. Y. Hellman, P. M. Muglia, Macromolecules, 4, 507 (1971)) in the Mark-Houwink equation:

$$\{N\} = KMa$$

where Kpp = 1.90E-04 , app = 0.725 and Kps = 1.26E-04, aps = 0.702.

In one embodiment of the invention, the thermoplastic resins utilized in the invention are characterized by a DSC curve with a Tme that remains essentially the same and a Tmax that decreases as the amount of unsaturated comonomer in the copolymer is increased. Tme means the temperature at which the melting ends and Tmax means the peak melting temperature, both as determined by one of ordinary skill in the art from DSC analysis using data from the final heating step. For such polymers, DSC analysis can be determined using a model Q1000 DSC from TA Instruments, Inc., which is calibrated using indium and deionized water.

In some embodiments of the present invention the copolymers are substantially free of diene-derived units. Dienes are nonconjugated diolefins which may be incorporated in polymers to facilitate chemical crosslinking reactions. "Substantially free of diene" is defined to be less than 1 % diene, or less than 0.5 % diene, or less than 0.1 % diene, or less than 0.05 % diene, or equal to 0 %. All of these percentages are by weight in the copolymer. The presence or absence of diene can be conventionally determined by infrared techniques well known to those skilled in the art.

Sources of diene include diene monomer added to the polymerization of ethylene and propylene, or use of diene in catalysts. No matter the source of such dienes, the above outlined limits on their inclusion in the copolymer are contemplated. Conjugated diene-containing metallocene catalysts have been suggested for the formation of copolymers of olefins. However, polymers made from such catalysts will incorporate the diene from the catalyst, consistent with the incorporation of other monomers in the polymerization.

In embodiments of our invention, a thermoplastic resin is included having a weight average molecular weight (Mw) of from 15,000-5,000,000, or from 20,000 to 1,000,000 and a molecular weight distribution Mw/Mn (sometimes referred to as a "polydispersity index" (PDI)) ranging from a lower limit of 1.01, 1.5 or 1.8 to an upper limit of 40 or 20 or 10 or 5 or 3.

Another measure of molecular weight typically used for polyethylene polymers is the melt index of the

polymer, also called $I_2$. The melt index is indirectly proportional to the molecular weight, although the relationship is not linear. For polyethylene the melt index is measured according to ASTM D-1238, condition 190 deg C./ 2.16 kg). Typical thermoplastic resins useful in embodiments of the invention have an $I_2$ in the range of from 0.001 to 1000 g/10 min. In some embodiments, the thermoplastic resin (A) has an I2 of from 0.5 to 500 g/10 min. Other embodiments include a thermoplastic resin with an I2 of from 1 to 300 g/10 min. The selection of suitable $I_2$ for the thermoplastic resin should be selected in view of the ease of melt kneadability and physical properties of the coating formed.

Melting Point and Crystallinity

Differential scanning calorimetry (DSC) is a common technique that can be used to examine the melting and crystallization of semi-crystalline polymers. General principles of DSC measurements and applications of DSC to studying semi-crystalline polymers are described in standard texts (e.g., E. A. Turi, ed., Thermal Characterization of Polymeric Materials, Academic Press, 1981). For example, DSC analysis may be determined using a model Q1000 DSC from TA Instruments, Inc, which is calibrated using indium and deionized water. After heating the sample rapidly to 230 °C and holding for 3 minutes, the cooling curve is obtained by cooling at 10 C/min to -40 °C. After holding at -40 °C for 3 minutes, the DSC melting endotherm is recorded while heating at 10 C/min. The melting point is determined using the standard TA DSC software.

In some embodiments, a primarily ethylene-based polyolefin is selected in which ethylene comprises from about 98 to 65 percent of polymer. Selected comonomer(s) make up the remainder of the polymer.

In some such embodiments, the ethylene polymer has the following characteristics and properties: 1) Crystallinity as determined by the observance of at least one endotherm when subjected to standard differential scanning calorimetry (DSC) evaluation; 2) a melt index of between about 30 and about 0.1 g/10 min, preferably of between 25 and 0.25 g/10 min, more preferably of between 22 and 0.5 g/10 min and most preferably of between 18 and 0.75 g/10 min; and 3) a density as determined according to ASTM D-792 of between about 0.845 and about 0.925 g/cc, preferably between 0.85 10 and 0.91 g/cc, and more preferably between 0.855 and 0.905 g/cc, and most preferably between 0.86 and 0.90 g/cc.

One class of resins particularly suited to use in embodiments of the invention are copolymers of ethylene and 1-octene or 1-butene, where ethylene comprises from about 90 to about 50, more preferably 85 to 55, and 1-octene or 1-butene from about 10 to about 50, more preferably about 15 to 45 percent by weight of the copolymer, and that have Melt Index of between about 0.25 and about 30, more preferably between 0.5 and 20 g/10 min. Alternatively, instead of a single Polymer a blend of polymers may be employed that has the physical characteristics described above. For example, it may be desirable to blend a first polymer with relatively high MI that is outside the range described above, with another of relatively low MI, so that the combined MI and the averaged density of the blend fall within the ranges noted above.

In addition to the thermoplastic resin, dispersions described herein include a dispersing agent. As used herein the term "dispersing agent" means an agent that aids in the formation and/or the stabilization of a dispersion. Some dispersing agents can also be used to form emulsions and are described in detail by Paul Becher (Emulsions: Theory and Practice, 3rd edition, Oxford University Press, New York, 2001), incorporated herein by reference in its entirety. Dispersing agents generally fall into three classes 1) surface-active materials, 2) naturally occurring materials, 3) finely divided solids. Surface-active agents, also called surfactants, are materials that reduce the interfacial tension between two immiscible liquid phases. They are classified according to the hydrophilic group in the molecule: anionic, cationic, nonionic, or ampholytic (amphoteric). Examples of commercially available dispersing agents is found in McCutcheon (McCutcheon's Emulsifiers and Detergents, Glen Rock, NJ, issued annually). Examples of naturally occurring materials include phospholipids, sterols, lanolin, water-soluble gums, alginates, carageenin, and cellulose derivatives. Examples of finely divided solids include basic salts of the metals, carbon black, powdered silica, and various clay (principally bentonite).

In some embodiments, a carboxylic acid or carboxylic acid salt is used as the dispersing agent. Typical salts include an alkali metal salt or an alkaline earth metal salts of the fatty acid. Other salts include ammonium or alkyl ammonium salts of the carboxylic acid. In some embodiments, the carboxylic acid or it's salt with 12 to fewer than 25 carbon atoms. Where the dispersing agent is a salt, the number of carbons refers to the carbon atoms associated with the carboxylic acid fragment. In other embodiments, the salt is formed with a fatty acid fragment that has at from 15 to 25 carbon atoms. Particular embodiments use an alkali metal salt of erucic acid. Erucic acid is a carboxylic acid with 22 carbon atoms. Some embodiments use erucic acid in the form of rapeseed oil which is a natural oil that contains approximately 40 to about 50% erucic acid with the remainder consisting of primarily chains having 18 carbon atoms. An alkali metal salt of erucic acid is also useful in some embodiments.

Some embodiments of the present invention use a fatty acid or its salt that is derived from an ester of a fatty acid. The alcohol residue constituting such ester may preferably contain 2 to 30 carbon atoms, and most preferably 6 to 20 carbon atoms. Such residue may be either a straight or a branched residue, and may also be a mixture of two or more residues each containing different number of carbon atoms. Exemplary such alcohol residues include residues of higher alcohols containing 10 to 20 carbon atoms such as cetyl alcohol, stearyl alcohol, and oleyl alcohol. Some embodiments use an ester wax of erucic acid.

In particular embodiments the salt of a fatty acid containing fewer than 25 carbon atoms is produced by neutralizing a fatty acid containing fewer than 25 carbon atoms or by saponification of an ester of a fatty acid containing fewer than 25 carbon atoms.

In other embodiments, the dispersing agent can be an ethlyene acrylic acid copolymer. Still other emobodimetns use alkyl ether carboxylates as the dispersing agent.

Embodiments of the aqueous dispersions described herein contain water in addition to the components as described above. Deionized water is typically preferred. In some embodiments, water with excess hardness can undesirably affect the formation of a suitable dispersion. Particularly water containing high levels of alkaline earth ions, such as $Ca^{2+}$, should be avoided. The term "dispersion" as used herein refers to a finely divided solid or liquid in a continuous liquid medium. An aqueous dispersion is a dispersion in which the continuous liquid medium is water. The term "dispersion" as used herein in connection with the compositions of the invention is intended to include within its scope both emulsions of essentially liquid materials, prepared employing the thermoplastic resin and the dispersing agent, and dispersions of solid particles. Such solid dispersions can be obtained, for example, by preparing an emulsion as previously described, and then causing the emulsion particle to solidify by various means. Thus, when the components are combined, some embodiments provide an aqueous dispersion wherein content of the dispersing agent is present in the range of from 0.5 to 30 parts by weight, and content of (C) water is in the range of 1 to 35% by weight per 100 parts by weight of the thermoplastic polymer; and total content of (A) and (B) is in the range of from 65 to 99% by weight. In particular embodiments, the dispersing agent ranges from 2 to 20 parts by weight based on 100 parts by weight of the polymer. In some embodiments, the amount of dispersing agent is less than about 4 percent by wt., based on the weight of the thermoplastic polymer. In some embodiments, the dispersing agent comprises from about 0.5 percent by wt. to about 3 percent by weight, based on the amount of the thermoplastic polymer used. In other embodiments, about 1.0 to about 3.0 weight percent of the dispersing agent are used. Embodiments having less than about 4 weight percent dispersing agent are preferred where the dispersing agent is a carboxylic acid.

One feature of some embodiments of the invention is that the dispersions have a small particle size. Typically the average particle size is less than about 5 μm. Some preferred dispersions have an average particle size of less than about 1.5 μm. In some embodiments, the upper limit on the average particle size is about 4.5 μm, 4.0 μm, 3.5 μm, 3.75 μm, 3.5 μm, 3.0 μm, 2.5 μm, 2.0 μm, 1.5 μm, 1.0 μm, 0.5 μm, or 0.1 μm. Some embodiments have a lower limit on the average particle size of about 0.05 μm, 0.7 μm, 0.1 μm, 0.5 μm, 1.0 μm, 1.5 μm, 2.0 μm, or 2.5 μm. Thus, some particular embodiments have, for example, an average particle size of from about 0.05 μm to about 1.5 μm. While in other embodiments, the particles in the dispersion have an average particle size of from about 0.5 μm to about 1.5 μm. For particles that are not spherical the diameter of the particle is the average of the long and short axes of the particle. Particle sizes can be measured on a Coulter LS230 light-scattering particle size analyzer or other suitable device.

Another parameter that characterizes particles in the dispersions is the particle size distribution, defined herein as the volume average particle diameter (Dv) divided by number average particle diameter (Dn). Some embodiments are characterized by a particle size distribution of less than or equal to about 2.0. In other embodiments, the dispersions have a particle size distribution of less than or equal to about less than 1.9, 1.7, or 1.5.

In yet another particular embodiment the aqueous dispersion has a concentration of the solid content including (A) thermoplastic resin is in the range of from 10 to 70%. Another measure of solids content is by volume. In some embodiments, the dispersion has a percent solids of less than about 74% by volume. Other dispersions have a solids content of from about 5% to about 74% by volume. In some embodiments, the dispersions have a percent solids of less than about 70% by volume, less than about 65% by volume, or ranging from about 5% to about 50% by volume.

One feature of some of the dispersions described herein is the pH, which can affect the uses for which dispersions are suitable. Typically, the dispersions have a pH of less than 12. Preferably, the pH ranges from about 5 to about 11.5, preferably from about 7 to about 11., more preferably from about 9 to about 11. However, dispersions having a lower limit of the pH of about 5, about 6, about 7, about 8, about 9, about 10, or about 11 are contemplated. Dispersions having an upper limit on the pH of about 6, about 7, about 8, about 9, about 10, about 11, or about 12 are contemplated.

While any method may be used, one convenience way to prepare the dispersions described herein is by melt-kneading. Any melt kneading means known in the art may be used. In some embodiments a kneader, a Banbury mixer, single-screw extruder, or a multi-screw extruder is used. The melt kneading may be conducted under the conditions which are typically used for melt kneading the thermoplastic resin (A). A process for producing the dispersions in accordance with the present invention is not particularly limited. One preferred process, for example, is a process comprises melt-kneading the above-mentioned components according to U.S. Pat. No. 5756659 and U.S. Patent Application No. 20010011118 . A preferred melt-kneading machine is, for example, a multi screw extruder having two or more screws, to which a kneading block can be added at any position of the screws. If desired, it is allowable that the extruder is provided with a first material-supplying inlet and a second material-supplying inlet, and further third and forth material-supplying inlets in this order from the upper stream to the down stream along the flow direction of a material to be kneaded. Further, if desired, a vacuum vent may be added at an optional position of the extruder. In some embodiments, the dispersion is first diluted to contain about 1 to about 3% by weight of water and then subsequently further diluted to comprise greater than 25% by weight of water. In some embodiments, the further dilution provides a dispersion with at least about 30% by weight of water. The aqueous dispersion obtained by the melt kneading may be further supplemented with an aqueous dispersion of an ethylene-vinyl compound copolymer, or a dispersing agent.

Figure 1 schematically illustrates such an extrusion apparatus embodiments of the invention. An extruder, in certain embodiments a twin screw extruder, 20 is coupled to a back pressure regulator, melt pump, or gear pump, 30. Embodiments also provide a base reservoir, 40 and an initial water reservoir 50, each of which includes a pump (not shown). Desired amounts of base and initial water are provided from the base reservoir 40 and the initial water reservoir 50, respectively. Any suitable pump may be used, but in some embodiments a pump that provides a flow of about 150 cc/min at a pressure of 240 bar is used to provide the base and the initial water to the extruder 20. In other embodiments, a liquid injection pump provides a flow of 300 cc/min at 200 bar or 600 cc/min at 133 bar. In some embodiments the base and initial water are preheated in a preheater.

Resin in the form of pellets, powder or flakes is fed from the feeder 80 to an inlet 90 of the extruder 20 where the resin is melted or compounded. In some embodiments, the dispersing agent is added to the resin through an along with the resin and in other embodiments, the dispersing agent is provided separately to the twin screw extruder 20. The resin melt is then delivered to from the mix and convey zone to an emulsification zone of the extruder where the initial amount of water and base from the reservoirs 40 and 50 is added through inlet 55. In some embodiments, dispersing agent may be added additionally or exclusively to the water stream. In some embodiments, the emulsified mixture is further diluted with additional water inlet 95 from reservoir 60 in a dilution and cooling zone of the extruder 20. Typically, the dispersion is diluted to at least 30 weight percent water in the cooling zone. In addition, the diluted mixture may be diluted any number of times until the desired dilution level is achieved. In some embodiments, water is not added into the twin screw extruder 20 but rather to a stream containing the resin melt after the melt has exited from the extruder. In this manner, steam pressure build-up in the extruder 20 is eliminated.

In some embodiments a basic substance or aqueous solution, dispersion or slurry thereof is added to the dispersion at any point of the process, preferably to the extruder. Typically the basic substance is added as an aqueous solution. But in some embodiments, it is added in other convenient forms, such as pellets or granules. In some embodiments, the basic substance and water are added through separate inlets of the extruder. Examples of the basic substance which may be used for the neutralization or the saponification in the melt kneading process include alkaline metals and alkaline earth metals such as sodium, potassium, calcium, strontium, barium;inorganic amines such as hydroxylamine or hydrazine; organic amines such as methylamine, ethylamine, ethanolamine, cyclohexylamine, tetramethylammonium hydroxide; oxide, hydroxide, and hydride of alkaline metals and alkaline earth metals such as sodium oxide, sodium peroxide, potassium oxide, potassium peroxide, calcium oxide, strontium oxide, barium oxide, sodium hydroxide, potassium hydroxide, calcium hydroxide, strontium hydride, barium hydroxide, sodium hydride, potassium hydride, calcium hydride; and weak acid salts of alkaline metals and alkaline earth metals such as sodium carbonate, potassium carbonate, sodium hydrogencarbonate, potassium hydrogencarbonate, calcium hydrogencarbonate, sodium acetate, potassium acetate, calcium acetate; or ammonium hydroxide. In particular embodiments, the basic substance is a hydroxide of an alkaline metal or a hydroxide of an alkali metal. In some embodiments, the basic substance is selected from potassium hydroxide, sodium hydroxide and combinations thereof.

The aqueous dispersion may be coated onto a substrate by various procedures, and for example, by spray coating, curtain flow coating, coating with a roll coater or a gravure coater, brush coating, dipping. The coating is preferably dried by heating the coated substrate to 50 to 150 °C for 1 to 300 seconds although the drying may be accomplished by any suitable means.

The substrate for coatings may comprise a film of a thermoplastic resin such as polyethylene terephthalate, polyethylene, polycarbonate, polyimide, polyamide, polyphenylene sulfide, polysulfone, aromatic polyester, polyether ether ketone, polyether sulfone, and polyether imide. The preferred is the film comprising polyethylene terephthalate, polyethylene, polyamide, and polycarbonate, and the most preferred is the film comprising polyethylene terephthalate. Typically the films have a thickness in the range of from 0.5 to 50 microns, although some have a thickness of from 1 to 30 microns.

Some embodiments of the dispersions described herein are capable of forming a coating which exhibits excellent water resistance, oil resistance, or chemical resistance. Some embodiments exhibit adhesion to non-polar materials, and therefore, when the aqueous dispersion of the present invention is coated and dried on the surface of a substrate such as paper, fiber, wood, metal, or plastic molded article, the resulting resin coating will provide the substrate with water resistance, oil resistance, chemical resistance, corrosion resistance and heat sealability. Coatings obtained from some dispersions described herein exhibit excellent moisture resistance, water repellency, thermal adhesion to paper, especially for water and/or grease barrier and ink adhesion coatings layers, metal, glass, wood, fiber (natural fiber and synthetic fiber), and nonwoven fabric, thermal transfer properties, abrasion resistance, impact resistance, weatherability, solvent resistance, flexibility, and adaptability to high-frequency fabricating. Some dispersions are particularly suited for the formation of textile coatings including fabric impregnation. Some dispersions are also suitable for use as carpet backing layers. Coatings for architectural works are also contemplated as well as coatings for controlled release coatings on fertilizer pellets or as coatings to control surface properties such as coefficient of friction. Additionally some dispersions can be used to prepare stable froths and foams, as described in "Froths and Durable Foams of Dispersed Olefin Polymers and articles Prepared from Same" filed concurrently herewith.

Some aqueous dispersions described herein are used as a binder in a coating composition for a coated wallpaper; a fiber coating agent (for improving the strength, moisture adsorption, or water repellency of the fiber); a net for construction, a sizing agent for nylon, polyester or glass fibers; a sizing agent/thermal adhesive of a paper or a nonwoven fabric; and an agent for imparting heat sealability with a paper or a film; a thermal adhesive of a sterilized paper; a binder of an ink or a coating composition; a surface coating agent for a paper or a film adapted for use with an ink jet printer; an agent for improving chipping resistance of an automotive coating composition; and the like.

In some embodiments, the aqueous dispersions have additional components in an amount that does not adversely affect the object of the present invention. Exemplary such additional components include water-soluble amino resins such as water-soluble melamine resin and water-soluble benzoguanamine resin and water-soluble epoxy resins for improving coating performance; organic thickeners such as polyvinyl alcohol, polyvinyl pyrrolidone, polyvinyl methylether, polyethylene oxide, polyacrylamide, polyacrylic acid, carboxy methyl cellulose, methyl cellulose, and hydroxyethyl cellulose and inorganic thickeners such as silicon dioxide, active clay, and bentonite for improving the stability and adjusting the viscosity of the dispersion; dispersing agents such as nonionic dispersing agents and anionic dispersing agents and water-soluble polyvalent metal salts for improving the stability of the dispersion; other additives such as anti-rust agent, anti-mold agent, UV absorber, thermal stabilizer, foaming agent, antifoaming agent, and the like; pigments such as titanium white, red iron oxide, phthalocyanine, carbon black, permanent yellow; and fillers such as calcium carbonate, magnesium carbonate, barium carbonate, talk, aluminum hydroxide, calcium sulfate, kaolin, mica, asbestos, mica, and calcium silicate.

EXAMPLES

Preparation Example 1

100 parts by weight of a thermoplastic ethylene-vinyl acetate commercially available from DuPont having a vinyl acetate content of about 28 wt %, a density of about 0.95 g/cm$^3$ (ASTM D-792) and a melt index of about 6 g/10 min. (as determined according to ASTM D1238 at 190 °C and 2.16 kg), and a melting point of about 73 °C (as determined according to ASTM D3417) and 4.2 parts by weight of a C32 carboxylic acid (Unicid 425 manufactured by Baker-Petrolite, acid value 97 mg KOH/g) are melt kneaded at 180 °C in twin screw extruder at a rate of 8.3 kg/hr.

Upon the melt kneaded resin/surfactant, 4.6 wt % aqueous solution of potassium hydroxide is continuously fed into a downstream injection port at a rate 0.9 kg/hr (at a rate of 10 wt % of the total mixture). This aqueous dispersion is subsequently diluted with additional water at a rate of 5.7 kg/hr before exiting the extruder. An aqueous dispersion having a solids content of 56 wt % at pH 10.7 is obtained. The dispersed polymer phase measured by a Coulter LS230 particle analyzer consisted of an average volume diameter of 0.56 micron and a particle size distribution (dv/dn) of 1.45.

Preparation Example 2

100 parts by weight of a thermoplastic ethylene/ 1-octene copolymer with an octene content of about 38 wt %, a density of about 0.87 g/cm$^3$ (ASTM D-792) and a melt index of about 5 g/10 min (as determined according to ASTM D1238 at 190 °C and 2.16 kg) a Mw/Mn of about 2.0, and a melting point of about 63 °C (as determined by DSC at a scanning rate of about 10 °C per minute), commercially available from DuPont Dow Elastomers, and 3.1 parts by weight of a C18/C16 carboxylic acid (Industrene 106 manufactured by CK Witco, acid value 200 mg KOH/g) are melt kneaded at 125 °C in twin screw extruder at a rate of 7.9 kg/hr.

Upon the melt kneaded resin/surfactant, 23.9 wt % aqueous solution of potassium hydroxide is continuously fed into a downstream injection port at a rate 0.2 kg/hr (at a rate of 2.5 wt % of the total mixture). This aqueous dispersion is subsequently diluted with additional water at a rate of 5.4 kg/hr before exiting the extruder. To further dilute the resulting dispersion, additional water is added at a rate of 0.7 kg/hr after the mixture exited the extruder. An aqueous dispersion having a solids content of 56 wt % at pH 9.6 is obtained. The dispersed polymer phase measured by a Coulter LS230 particle analyzer consisted of an average volume diameter of 2.04 micron and a particle size distribution (dv/dn) of 1.18.

Preparation Example 3

100 parts by weight of a thermoplastic ethylene/1-octene copolymer with octene content of about 38 wt %, a density of about 0.87 g/cm3 (ASTM D-792) and a melt index of about 5 g/10 min. (as determined according to ASTM D1238 at 190 °C and 2.16 kg) a Mw/Mn of about 2.0, and a melting point of about 63 °C (as determined by DSC at a scanning rate of about 10 °C per minute.), commercially available from DuPont Dow Elastomers, and 3.6 parts by weight of a C22/C18 carboxylic acid (High-erucic rapeseed oil manufactured by Montana Specialty Mills, acid value 97 mg KOH/g) are melt kneaded at 150 °C in twin screw extruder at a rate of 5.0 kg/hr.

Upon the melt kneaded resin/surfactant, 16.3 wt % aqueous solution of potassium hydroxide is continuously fed into a downstream injection port at a rate 0.1 kg/hr (at a rate of 2.0 wt % of the total mixture). This aqueous dispersion is subsequently diluted with additional water at a rate of 3.2 kg/hr before exiting the extruder. To further dilute the resulting dispersion, additional water is added at a rate of 0.8 kg/hr after the mixture exited the extruder. An aqueous dispersion having a solids content of 55 wt % at pH 10.7 is obtained. The dispersed polymer phase measured by a Coulter LS230 particle analyzer consisted of an average volume diameter of 1.11 micron and a particle size distribution (dv/dn) of 1.85.

Preparation Example 4

100 parts by weight of a thermoplastic ethylene/ 1-octene copolymer with octene content of about 38 wt %, a density of about 0.87 g/cm$^3$ (ASTM D-792) and a melt index of about 5 g/10 min. (as determined according to ASTM D1238 at 190 °C and 2.16 kg) a Mw/Mn of about 2.0, and a melting point of about 63 °C (as determined by DSC at a scanning rate of about 10 °C per minute.), commercially available from DuPont Dow Elastomers and 3.1 parts by weight of a C26 carboxylic acid (Unicid 350 manufactured by Baker-Petrolite, acid value 115 mg KOH/g) are melt kneaded at 150 °C in twin screw extruder at a rate of 9.7 kg/hr.

Upon the melt kneaded resin/surfactant, 12.5 wt % aqueous solution of potassium hydroxide is continuously fed into a downstream injection port at a rate 0.2 kg/hr (at a rate of 2.0 wt % of the total mixture). This aqueous dispersion is subsequently diluted with additional water at a rate of 7.5 kg/hr before exiting the extruder. An aqueous dispersion having a solids content of 56 wt % at pH 10.8 is obtained. The dispersed polymer phase measured by a Coulter LS230 particle analyzer consisted of an average volume diameter of 0.79 micron and a particle size distribution (dv/dn) of 1.95.

As demonstrated above, the invention provides new dispersions useful for many applications. In some instances, the new have one or more of the following advantages. First, some new dispersions have better durability. Certain dispersions exhibit improved adhesion properties and others may have improved adhesion as well as good toughness and durability. Other dispersions are easier to process in a melt-extrusion process. In particular, some dispersions are easier to process due to the low melting point of the polymers used therein. Some dispersions have the feature of being low yellowing. Other characteristics and additional advantages are apparent to those skilled in the art.

While the invention has been described with respect to a limited number of embodiments, the specific features of one embodiment should not be attributed to other embodiments of the invention. No single embodiment is representative of all aspects of the inventions. Moreover, variations and modifications therefrom exist. For example, the dispersions described herein may comprise other components. Various additives may also be used to further enhance one or more properties. In some embodiments, the dispersions are substantially free of any additive not specifically enumerated herein. Some embodiments of the dispersions described herein consist of or consist essentially of the enumerated components. In addition, some embodiments of the methods described herein consist of or consist essentially of the enumerated steps. The appended claims intend to cover all such variations and modifications as falling within the scope of the invention.

## Claims

1. An aqueous dispersion comprising

 (A) at least one thermoplastic resin;
 (B) at least one dispersing agent, comprising at least one carboxylic acid, a salt of at least one carboxylic acid, a carboxylic acid ester or salt of a carboxylic acid ester, or an ethylene acid polymer;
 (C) water; and optionally
 (D) a neutralizing agent selected from potassium hydroxide, sodium hydroxide and combinations thereof;

 wherein the dispersion has a pH of from 7 to 11, and wherein the thermoplastic resin is selected from an alpha-olefin interpolymer of ethylene with at least one comonomer selected from propylene, 1-butene, 3-methyl-1-butene, 4-methyl-1-pentene, 3-methyl-1-pentene, 1-heptene, 1-hexene, 1-octene, 1-decene, and 1-dodecene, wherein the dispersion has a particle size distribution (defined as volume average particle diameter Dv divided by number average particle diameter Dn) of less than or equal to 2.0, and wherein the dispersing agent comprises less than about 4 percent by weight based on the weight of the thermoplastic resin.

2. An aqueous dispersion according to Claim 1, wherein the dispersion has a volume average particle size of less than 5 $\mu$m.

3. An aqueous dispersion according to Claim 1, wherein the dispersing agent comprises at least one carboxylic acid, a salt of at least one carboxylic acid, a carboxylic acid ester or salt of the carboxylic acid ester, or is selected from ethylene carboxylic acid polymers and their salts, alkyl ether carboxylates, petroleum sulfonates, sulfonated polyoxyethylenated alcohol, sulfated or phosphated polyoxyethylenated alcohols, polymeric ethylene oxide/propylene oxide/ethylene oxide dispersing agents, primary and secondary alcohol ethoxylates, alkyl glycosides and alkyl glycerides.

4. An aqueous dispersion according to Claim 1, wherein the dispersing agent is selected from ethylene acrylic acid copolymers or ethylene methacrylic acid copolymers.

5. An aqueous dispersion according to Claim 1, wherein the dispersing agent comprises at least one carboxylic acid.

6. An aqueous dispersion according to Claim 5, wherein the carboxylic acid has from 12 to 60 carbon atoms.

7. An aqueous dispersion according to Claim 1, wherein the dispersing agent comprises less than 4 percent by weight, based on the weight of the thermoplastic resin.

8. An aqueous dispersion according to Claim 1, wherein the dispersion has a particle size distribution (Dv/Dn) of less than or equal to 1.5.

9. An aqueous dispersion according to Claim 1, wherein the dispersion has a volume average particle size of from 0.05 $\mu$m to 1.5 $\mu$m.

10. An aqueous dispersion according to Claim 1, wherein the dispersion has a solids content of from 5% to 74% by volume

11. A method for producing an aqueous dispersion comprising:

(1) melt kneading (A) at least one thermoplastic resin; and (B) at least one dispersing agent, to produce a melt-kneaded product,
(2) diluting said melt-kneaded product, and melt kneading the resulting mixture to form the aqueous dispersion;

wherein:-
the thermoplastic resin is selected from an alpha-olefin interpolymer of ethylene with at least one comonomer selected from propylene, 1-butene, 3-methyl-1-butene, 4-methyl-1-pentene, 3-methyl-1-pentene, 1-heptene, 1-hexene, 1-octene, 1-decene, and 1-dodecene;
the dispersing agent comprising at least one carboxylic acid, a salt of at least one carboxylic acid, a carboxylic acid ester or salt of a carboxylic acid ester, or an ethylene acid polymer;
whereby the resulting dispersion has a particle size distribution (defined as volume average particle diameter Dv divided by number average particle diameter Dn) of less than or equal to 2.0, and a pH of from 7 to 11.

**Patentansprüche**

1. Eine wässrige Dispersion, die Folgendes beinhaltet:

(A) mindestens einen Thermoplast;
(B) mindestens ein Dispergiermittel, das mindestens eine Carbonsäure, ein Salz von mindestens einer Carbonsäure, einen Carbonsäureester oder das Salz eines Carbonsäureesters oder ein Ethylen-Säure-Polymer beinhaltet;
(C) Wasser; und optional
(D) ein Neutralisationsmittel, ausgewählt aus Kaliumhydroxid, Natriumhydroxid und Kombinationen daraus;

wobei die Dispersion einen pH-Wert von 7 bis 11 aufweist, und
wobei der Thermoplast aus einem Alpha-Olefin-Interpolymer von Ethylen mit mindestens einem Comonomer, ausgewählt aus Propylen, 1-Buten, 3-Methyl-1-buten, 4-Methyl-1-penten, 3-Methyl-1-penten, 1-Hepten, 1-Hexen, 1-Octen, 1-Decen und 1-Dodecen, ausgewählt ist,
wobei die Dispersion eine Partikelgrößenverteilung (definiert als Volumen durchschnittlicher Partikeldurchmesser Dv geteilt durch die Partikeldurchmesserdurchschnittszahl Dn) von weniger als oder gleich 2,0 aufweist, und wobei das Dispergiermittel weniger als etwa 4 Gewichtsprozent basierend auf dem Gewicht des Thermoplasten beinhaltet.

2. Wässrige Dispersion gemäß Anspruch 1, wobei die Dispersion eine durchschnittliche Volumen-Partikelgröße von weniger als 5 $\mu$m aufweist.

3. Wässrige Dispersion gemäß Anspruch 1, wobei das Dispergiermittel mindestens eine Carbonsäure, ein Salz von mindestens einer Carbonsäure, einen Carbonsäureester oder das Salz des Carbonsäureesters beinhaltet oder aus Ethylencarbonsäurepolymeren und ihren Salzen, Alkylethercarboxylaten, Petroleumsulfonaten, sulfoniertem polyoxyethyliniertem Alkohol, sulfatierten oder phosphatierten polyoxyethylenierten Alkoholen, polymeren Ethylenoxid-/Propylenoxid-/Ethylenoxiddispergiermitteln, primären und sekundären Alkohol-Ethoxylaten, Alkylglykosiden und Alkylglyceriden ausgewählt ist.

4. Wässrige Dispersion gemäß Anspruch 1, wobei das Dispergiermittel aus Ethylen-Acrylsäurecopolymeren oder Ethylen-Methacrylsäurecopolymeren ausgewählt ist.

5. Wässrige Dispersion gemäß Anspruch 1, wobei das Dispergiermittel mindestens eine Carbonsäure beinhaltet.

6. Wässrige Dispersion gemäß Anspruch 5, wobei die Carbonsäure von 12 bis 60 Kohlenstoffatome aufweist.

7. Wässrige Dispersion gemäß Anspruch 1, wobei das Dispergiermittel, bezogen auf das Gewicht des Thermoplasts, weniger als 4 Gewichtsprozent beinhaltet.

8. Wässrige Dispersion gemäß Anspruch 1, wobei die Dispersion eine Partikelgrößenverteilung (Dv/Dn) von weniger als oder gleich 1,5 aufweist.

9. Wässrige Dispersion gemäß Anspruch 1, wobei die Dispersion eine durchschnittliche Volumen-Partikelgröße von 0,05 $\mu$m bis 1,5 $\mu$m aufweist.

10. Wässrige Dispersion gemäß Anspruch 1, wobei die Dispersion einen Festkörperanteil von 5 bis 74 Vol.-% aufweist.

11. Ein Verfahren zum Produzieren einer wässrigen Dispersion, das Folgendes beinhaltet:

(1) Schmelzkneten (A) von mindestens einem Thermoplast; und (B) mindestens ein Dispergiermittel, um ein schmelzgeknetetes Produkt zu produzieren,

(2) Verdünnen des schmelzgekneteten Produkts, und Schmelzkneten der resultierenden Mischung, um die wässrige Dispersion zu bilden;

wobei:

der Thermoplast aus einem Alpha-Olefin-Interpolymer von Ethylen mit mindestens einem Comonomer, ausgewählt aus Propylen, 1-Buten, 3-Methyl-1-buten, 4-Methyl-1-penten, 3-Methyl-1-penten, 1-Hepten, 1-Hexen, 1-Octen, 1-Decen und 1-Dodecen, ausgewählt ist; das Dispergiermittel mindestens eine Carbonsäure, ein Salz von mindestens einer Carbonsäure, einen Carbonsäureester oder das Salz eines Carbonsäureesters oder ein Ethylen-Säure-Polymer beinhaltet; wobei die resultierende Dispersion eine Partikelgrößenverteilung (definiert als Volumen durchschnittlicher Partikeldurchmesser Dv geteilt durch die Partikeldurchmesserdurchschnittszahl Dn) von weniger als oder gleich 2,0 und einen pH-Wert von 7 bis 11 aufweist.

## Revendications

1. Une dispersion aqueuse comprenant

(A) au moins une résine thermoplastique;
(B) au moins un agent dispersant comprenant au moins un acide carboxylique, un sel d'au moins un acide carboxylique, un ester d'acide carboxylique ou un sel d'un ester d'acide carboxylique, ou un polymère d'acide éthylénique ;
(C) de l'eau ; et facultativement
(D) un agent neutralisant sélectionné parmi l'hydroxyde de potassium, l'hydroxyde de sodium et des combinaisons de ceux-ci ;

la dispersion ayant un pH allant de 7 à 11, et dans laquelle la résine thermoplastique est sélectionnée parmi un interpolymère d'alpha-oléfine d'éthylène avec au moins un comonomère sélectionné parmi le propylène, le 1-butène, le 3-méthyl-1-butène, le 4-méthyl-1-pentène, le 3-méthyl-1-pentène, le 1-heptène, le 1-hexène, le 1-octène, le 1-décène, et le 1-dodécène, la dispersion ayant une répartition granulométrique (définie comme étant le diamètre de particule moyen en volume Dv divisé par le diamètre de particule moyen en nombre Dn) inférieure ou égale à 2,0, et dans laquelle l'agent dispersant constitue en moins d'environ 4 pour cent en poids rapporté au poids de la résine thermoplastique.

2. Une dispersion aqueuse selon la revendication 1, la dispersion ayant une taille de particule moyenne en volume inférieure à 5 $\mu$m.

3. Une dispersion aqueuse selon la revendication 1, dans laquelle l'agent dispersant comprend au moins un acide carboxylique, un sel d'au moins un acide carboxylique, un ester d'acide carboxylique ou un sel de l'ester d'acide carboxylique, ou est sélectionné parmi des polymères d'éthylène et d'acide carboxylique et leurs sels, des carboxylates d'éther d'alkyle, des sulfonates de pétrole, un alcool polyoxyéthyléné sulfoné, des alcools polyoxyéthylénés sulfatés ou phosphatés, des agents dispersants oxyde d'éthylène/oxyde de propylène/oxyde d'éthylène polymères, des éthoxylates d'alcools primaires et secondaires, des glycosides d'alkyle et des glycérides d'alkyle.

**4.** Une dispersion aqueuse selon la revendication 1, dans laquelle l'agent dispersant est sélectionné parmi des copolymères d'éthylène et d'acide acrylique ou des copolymères d'éthylène et d'acide méthacrylique.

**5.** Une dispersion aqueuse selon la revendication 1, dans laquelle l'agent dispersant comprend au moins un acide carboxylique.

**6.** Une dispersion aqueuse selon la revendication 5, dans laquelle l'acide carboxylique a de 12 à 60 atomes de carbone.

**7.** Une dispersion aqueuse selon la revendication 1, dans laquelle l'agent dispersant constitue moins de 4 pour cent en poids, rapporté au poids de la résine thermoplastique.

**8.** Une dispersion aqueuse selon la revendication 1, la dispersion ayant une répartition granulométrique (Dv/Dn) inférieure ou égale à 1,5.

**9.** Une dispersion aqueuse selon la revendication 1, la dispersion ayant une taille de particule moyenne en volume allant de 0,05 $\mu$m à 1,5 $\mu$m.

**10.** Une dispersion aqueuse selon la revendication 1, la dispersion ayant une teneur en solides allant de 5 % à 74 % en volume.

**11.** Une méthode pour produire une dispersion aqueuse comprenant les étapes consistant à :

(1) pétrir à chaud (A) au moins une résine thermoplastique ; et (B) au moins un agent dispersant, pour produire un produit pétri à chaud, (2) diluer ledit produit pétri à chaud, et pétrir à chaud le mélange résultant pour former la dispersion aqueuse ;

dans laquelle :

la résine thermoplastique est sélectionnée parmi un interpolymère d'alpha-oléfine d'éthylène avec au moins un comonomère sélectionné parmi le propylène, le 1-butène, le 3-méthyl-1-butène, le 4-méthyl-1-pentène, le 3-méthyl-1-pentène, le 1-heptène, le 1-hexène, le 1-octène, le 1-décène, et le 1-dodécène ; l'agent dispersant comprenant au moins un acide carboxylique, un sel d'au moins un acide carboxylique, un ester d'acide carboxylique ou un sel d'un ester d'acide carboxylique, ou un polymère d'acide éthylénique ; grâce à quoi la dispersion résultante a une répartition granulométrique (définie comme étant le diamètre de particule moyen en volume Dv divisé par le diamètre de particule moyen en nombre Dn) inférieure ou égale à 2,0, et un pH allant de 7 à 11.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5756659 A

- US 20010011118 A

### Non-patent literature cited in the description

- **RANDALL.** Journal of Macromolecular Science. *Macromolecular Chemistry and Physics,* 1989, vol. C29, 201-317
- **TH.G. SCHOLTE ; N.L.J. MEIJERINK ; H.M. SCHOFFELEERS ; A.M.G. BRANDS.** *J. Appl. Polym. Sci.,* 1984, vol. 29, 3763-3782

- **E. P. OTOCKA ; R. J. ROE ; N. Y. HELLMAN ; P. M. MUGLIA.** *Macromolecules,* 1971, vol. 4, 507
- Thermal Characterization of Polymeric Materials. Academic Press, 1981
- **PAUL BECHER.** Emulsions: Theory and Practice. Oxford University Press, 2001